# FASCICULE DE BREVET EUROPEEN

(11) **EP 2 934 417 B1**
(45) Date de publication et mention de la délivrance du brevet: **20.09.2017**
(21) Numéro de dépôt: 13815759.9
(22) Date de dépôt: 20.12.2013
(51) Int. Cl.: A61B 46/00

(54) **EMBALLAGE PRIMAIRE POUR DISPOSITIF MÉDICAL STÉRILE**
PRIMÄRVERPACKUNG FÜR EINE STERILE MEDIZINISCHE VORRICHTUNG
PRIMARY PACKAGING FOR A STERILE MEDICAL DEVICE

(30) Priorité: 20.12.2012 FR 1262468
(43) Date de publication de la demande: 28.10.2015
(73) Titulaire: Peters Surgical, 93000 Bobigny (FR)
(72) Inventeur: CAMEDDA, Caroline, F-95410 Groslay (FR); VAN LANDEGHEM, Nathalie, F-75011 Paris (FR)
(74) Mandataire: Regimbeau
(86) Numéro de dépôt international: PCT/EP2013/077862
(87) Numéro de publication internationale: WO 2014/096434

(56) Documents cités:
- US-A- 4 342 392
- US-A- 4 615 435

## Description

### DOMAINE DE L'INVENTION

L'invention se rapporte au domaine des emballages de matériel à usage médical.

Elle peut concerner de nombreuses applications à usage médical. Cependant, sans que cela soit limitatif, elle s'applique en particulier aux mèches de gaze ou tricots utilisés en chirurgie.

### ART ANTERIEUR

De nombreux types d'emballage prévus pour contenir du matériel à usage médical ont déjà été proposés, voir par exemple les documents US4615435 et us4342392. Pour diminuer les risques de contamination, le matériel chirurgical est généralement conservé dans des emballages aseptiques. Cependant, une fois un tel emballage ouvert, le matériel contenu ne se trouve plus en milieu aseptique.

Dans certains cas, le praticien se charge lui-même d'ouvrir l'emballage avant de saisir et utiliser le matériel médical qu'il renferme.

Dans d'autres cas, au cours d'une opération de chirurgie par exemple, les emballages sont d'abord ouverts par le personnel médical, et leur contenu est ensuite présenté au praticien qui peut alors se saisir du contenu.

Dans tous les cas, l'ouverture de l'emballage puis l'extraction de son contenu doit requérir un nombre minimal de manipulations de l'emballage lui-même, ces manipulations devant être opérées en garantissant le caractère stérile du contenu.

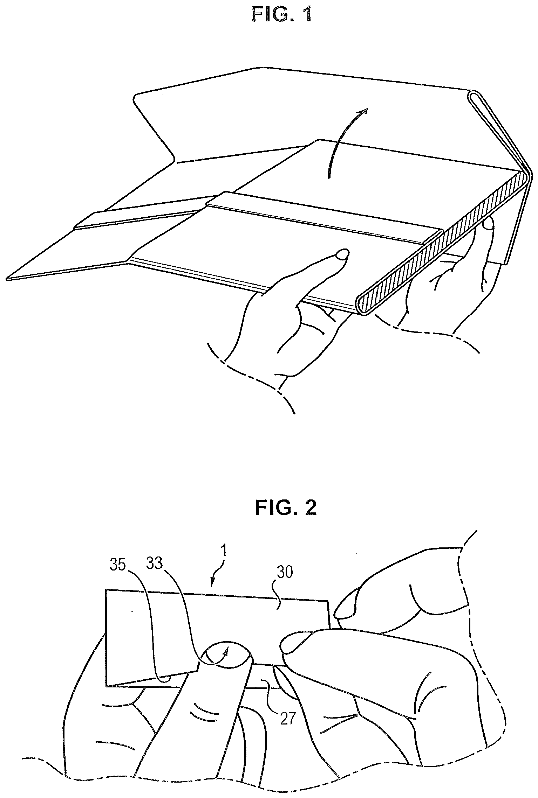

Un exemple d'emballage connu est décrit dans le document US 4,342,392. Ce document décrit un emballage réalisé par pliage d'une feuille de sorte à former un berceau de réception du contenu, formé de deux volets articulés entre eux en forme de pince, un élément de couverture articulé sur l'un des volets du berceau et des moyens de fermeture eux-mêmes articulés transversalement sur les volets du berceau et sur l'élément de couverture. Les moyens de fermeture sont fixés par collage. On a illustré schématiquement sur la figure 1 annexée, une étape de l'ouverture d'un tel emballage après séparation des moyens de fermeture latéraux et déploiement de ceux-ci.

L'examen de la figure 1 annexée montre que l'utilisation d'un tel emballage ne donne pas totalement satisfaction dans la mesure où l'ouverture est délicate car elle doit être réalisée partiellement en aveugle, l'une des mains de l'opérateur tenant le berceau en forme de pince qui loge le contenu à prélever, tandis que l'autre main doit être positionnée sous l'emballage afin de saisir l'élément de couverture pour déployer celui-ci.

### PRESENTATION DE L'INVENTION

L'invention vise à améliorer les techniques connues de l'art antérieur. L'invention vise en particulier à proposer un nouvel emballage qui tout en étant de réalisation simple et fiable, permet un accès facile et sûr au contenu, sans surcoût particulier.

Ce but est atteint selon la présente invention grâce à un emballage pour matériel médical réalisé par pliage d'une feuille de sorte à former un berceau de réception du contenu, formé de deux volets articulés entre eux en forme de pince, un élément de couverture articulé sur l'un des volets du berceau et des moyens de fermeture eux-mêmes articulés transversalement sur l'un au moins des volets du berceau et/ou de l'élément de couverture, caractérisé par le fait que les moyens de fermeture sont adaptés pour être fixés par simple engagement mécanique entre eux et que l'élément de couverture exerce, lors de sa sollicitation à l'ouverture, un effort sur lesdits moyens de fermeture engagés mécaniquement entre eux, assurant leur séparation automatique et de là l'ouverture de l'emballage.

Un avantage de la présente invention est que l'ouverture de l'emballage est réalisée en un geste manuel unique, instinctif, rapide, et sans risque pour le manipulateur de toucher directement le contenu de l'emballage. Un autre avantage réside dans le fait qu'aucun moyen supplémentaire de fixation tel que colle, adhésif ou autre n'est requis pour fermer l'emballage.

L'invention est avantageusement complétée par les caractéristiques suivantes, prises seules ou en une quelconque de leurs combinaisons techniquement possibles :
- au moins un des moyens de fermeture est articulé transversalement sur l'élément de couverture par une ligne de pliage transversale,
- au moins un des moyens de fermeture est un moyen de fermeture moteur articulé transversalement sur un des volets du berceau et sur l'élément de couverture par une ligne de pliage transversale motrice,
- l'élément de couverture est articulé à un volet d'embase du berceau par une ligne de pliage longitudinale sécante avec chaque ligne de pliage transversale,
- chaque moyen de fermeture moteur est traversé par un prolongement de la ligne de pliage longitudinale de l'élément de couverture sur le volet d'embase, lui permettant de former un dièdre lorsque l'élément de couverture est en position repliée,
- l'emballage comporte deux moyens de fermeture dont le premier au moins est un moyen de fermeture moteur en forme de dièdre, l'engagement mécanique entre eux dans leur position repliée étant réalisé par logement d'une portion du second moyen de fermeture à l'intérieur du dièdre formé par le premier moyen de fermeture moteur,
- les deux moyens de fermetures sont moteurs et en forme de dièdre, les deux lignes de pliage transversales motrices sont perpendiculaires à la ligne de pliage longitudinale de l'élément de couverture sur le volet d'embase, et les arêtes des dièdres se touchent en au moins un segment lorsque les moyens de fermeture sont engagés l'un dans l'autre dans leur position repliée,
- l'élément de couverture comporte un volet de couverture comprenant un bord libre opposé à une ligne de pliage longitudinale, dans lequel est formée au moins une échancrure laissant découverte une portion de surface du berceau lorsque l'élément de couverture est en position repliée,
- l'élément de couverture comporte un volet de couverture comprenant au moins un bord transversal comportant une portion transversale connectée à un moyen de fermeture et une portion transversale libre adjacente à un bord libre,
- les moyens de fermeture sont repliés contre le berceau et le recouvrent partiellement de sorte à laisser découverte une portion de surface sur le berceau,
- le berceau comprend un volet de protection replié sur un volet d'embase par une ligne de pliage de protection, les deux portions de surface laissées découvertes sur le berceau s'étendant de part et d'autre de cette ligne de pliage de protection de sorte à pouvoir être saisies simultanément par pinçage,
- au moins un moyen de fermeture moteur est symétrique par rapport au prolongement d'une ligne de pliage longitudinale qui le traverse, de manière à faire coïncider les deux faces du dièdre qu'il forme lorsque l'élément de couverture est en position repliée,
- l'emballage est réalisé en un matériau textile non-tissé, de préférence en fibres de polyéthylène.

### DESCRIPTION DES FIGURES

La figure 1 montre l'emballage connu déjà décrit.
La figure 2 présente un mode de réalisation préféré de l'invention vu du dessus et manipulé manuellement.
La figure 3 présente une ébauche du même mode de réalisation en vue du dessus avant mise en forme par pliage.
Les figures 4 à 7 présentent différentes étapes de préparation d'un emballage selon le mode de réalisation illustré en figure 3 :

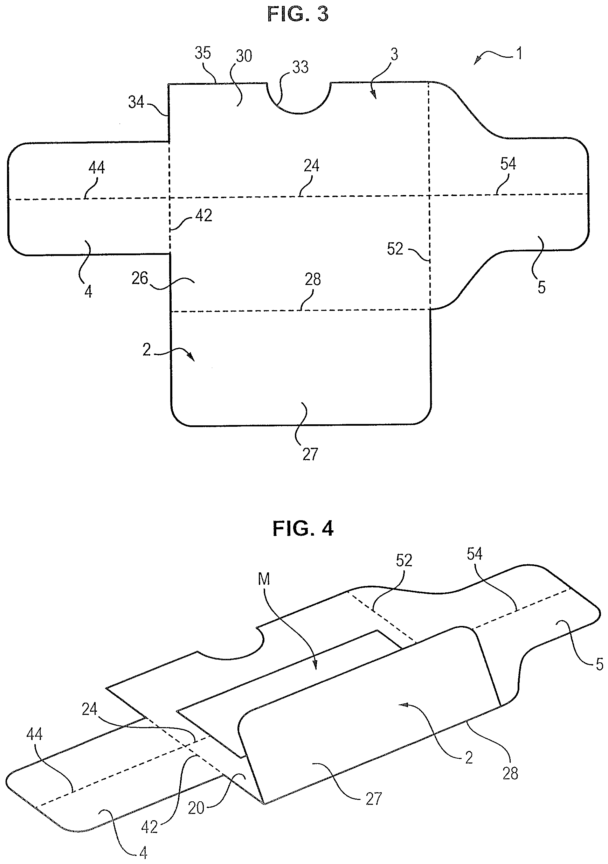

   - la figure 4 est une vue de face en perspective de l'emballage précité, dont le berceau de réception est en cours de mise en forme ;

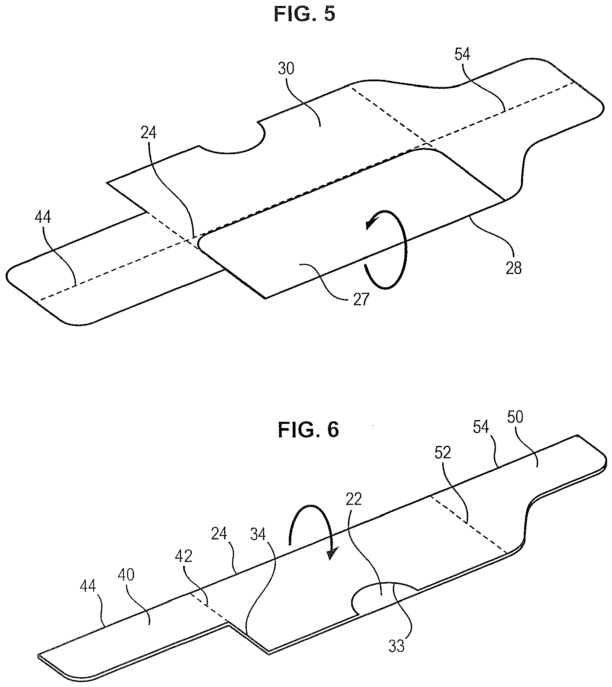

   - la figure 5 est une vue de face en perspective de l'emballage précité, dont la mise en forme du berceau de réception est achevée, avant pliage de l'élément de couverture ;
   - la figure 6 présente une vue de face en perspective de l'emballage précité, dont l'élément de couverture est en position repliée ;

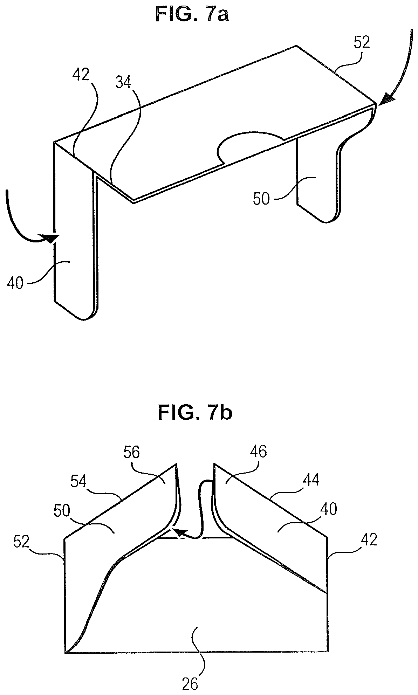

   - les figure 7a et 7b présentent respectivement une vue de face en perspective et une vue de dos de l'emballage précité dont les moyens de fermeture sont en cours de fermeture ;

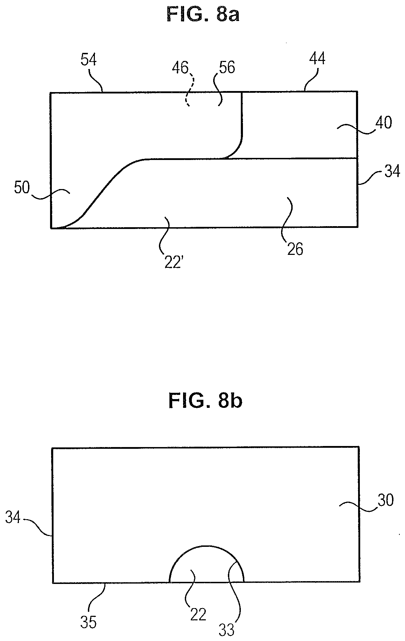

Les figure 8a et 8b présentent respectivement une vue de dos et de face de l'emballage précité en position fermée.

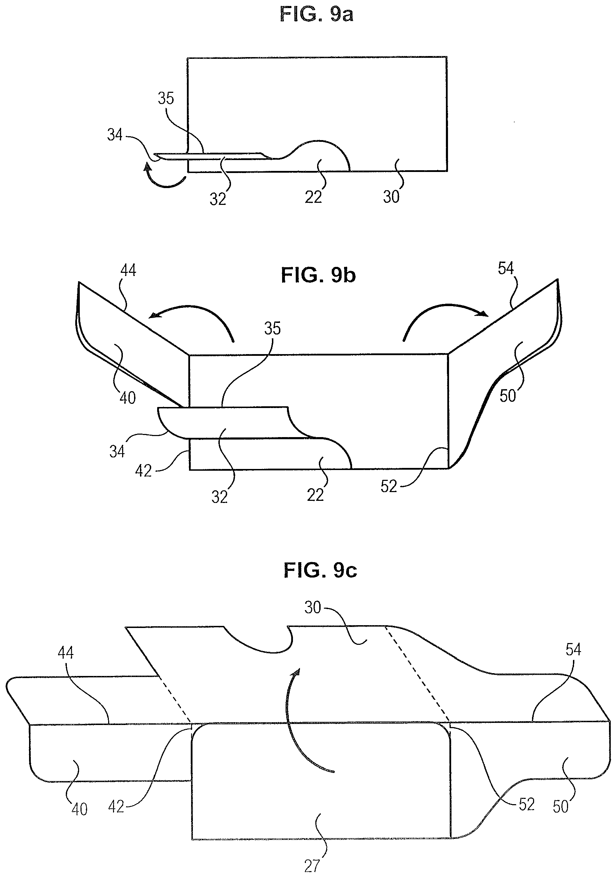

Les figures 9a à 9c présentent différents états d'un emballage selon le mode de réalisation de la figure 3 en cours d'ouverture :
   - La figure 9a présente une vue de face de l'emballage précité, dans lequel l'élément de couverture est sollicité ;
   - La figure 9b présente une vue de face de l'emballage précité, dont les moyens de fermeture sont en cours de dépliage ;
   - La figure 9c présente une vue de face en perspective de l'emballage précité, en position ouverte, autorisant l'accès à son contenu.

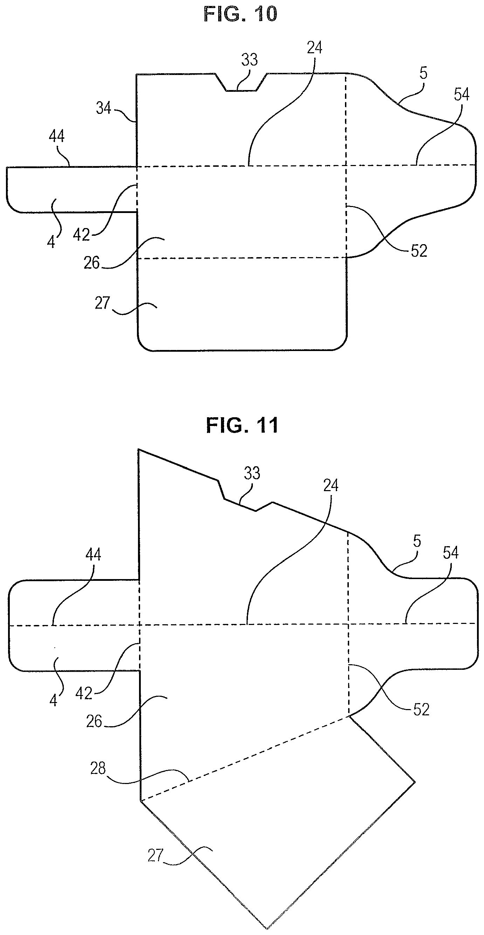

Les figures 10 à 17 présente des variantes de réalisation d'emballage selon l'invention :
   - Les variantes illustrées sur les figures 10 à 13 sont des ébauches en vue de dessus, avant leur mise en forme ;

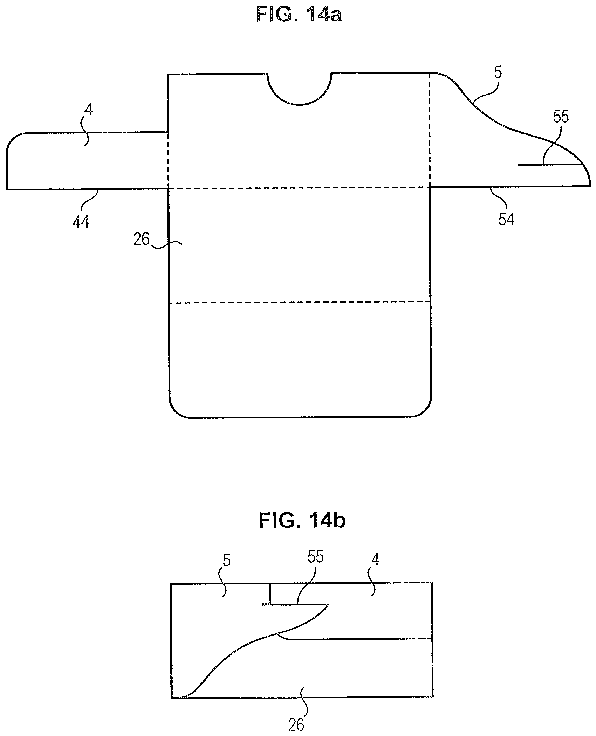

   - Les figures 14a et 14b présentent une même variante de réalisation, respectivement en vue de dessus avant mise en forme, et en vue de dos après mise en forme ;

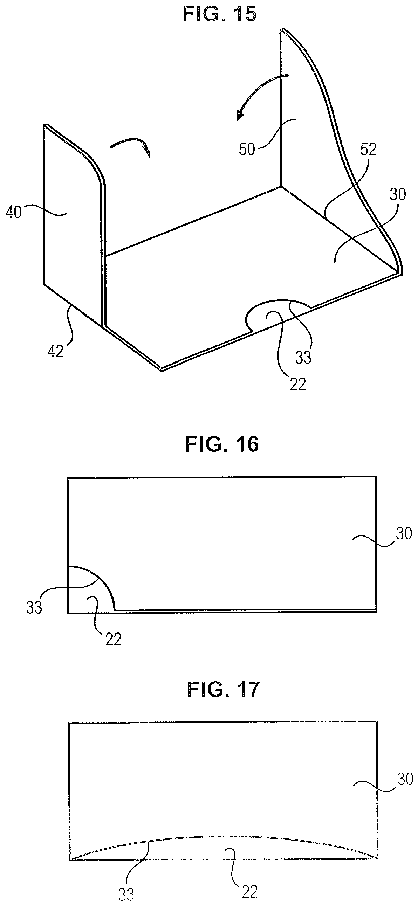

   - La figure 15 illustre une variante de réalisation en cours de pliage selon une vue en perspective de dessus ;
   - Les figures 16 et 17 présentent des variantes en vue de dessus.

### DESCRIPTION DETAILLEE DE L'INVENTION

Sera détaillé dans un premier temps un mode de réalisation préféré, illustré sur les figures 2 à 9c.

L'emballage 1 est réalisé à partir d'une feuille découpée et pliée pour constituer plusieurs parties assurant des fonctions distinctes et illustrées en figure 3 : un berceau de réception 2 destiné à recevoir et isoler un contenu M de tout contact extérieur, un élément de couverture 3 mobile par rapport au berceau de réception, et permettant de clore le berceau de réception, et des moyens de fermeture 4, 5 aptes à maintenir l'emballage fermé.

Le berceau de réception 2 peut comporter deux volets : un volet d'embase 26 et un volet de protection 27 articulé au volet d'embase 26 par une ligne de pliage de protection 28 permettant au berceau de réception de former une pince. Les volets d'embase 26 et de protection 27 sont de contours sensiblement identiques, avantageusement rectangulaires, la ligne de pliage de protection 28 étant placée sur un de leurs grands côtés. Le cas échéant le volet de protection 27 a ses deux angles opposés à la ligne de pliage de protection 28 arrondis. Par ailleurs, le volet de protection 27 peut posséder une largeur, considérée perpendiculairement à la ligne de pliage 28, inférieure à celle du volet d'embase 26. Une fois replié autour de la ligne de pliage 28, le volet de protection 27 recouvre le volet d'embase 26, le contenu M à protéger étant placé dans un logement 20 formé entre le volet d'embase 26 et le volet de protection 27.

L'élément de couverture 3 est articulé au volet d'embase 26 du berceau de réception 2 selon une ligne de pliage longitudinale 24 située sur un bord opposé à celui de la ligne de pliage de protection 28, et de préférence parallèle à celle-ci.

L'élément de couverture 3 comprend un volet de couverture 30, qui lui-même comporte un bord libre 35 opposé au bord où se trouve la ligne de pliage longitudinale 24.

Le volet de couverture 30 a de préférence un contour sensiblement identique à celui du volet d'embase 26, avantageusement rectangulaire, comme par exemple dans le mode de réalisation illustré en figure 3. Une fois replié autour de la ligne de pliage 24, le volet de couverture 30 recouvre le volet de protection 27 et par conséquent le berceau de réception 2 recevant le contenu M.

Cependant, le bord libre 35 du volet de couverture 30 n'est de préférence pas rectiligne mais présente une concavité 33 permettant un accès au berceau 2 sous-jacent. Par exemple, le volet de couverture 30 est creusé sur son bord libre 35 d'au moins une échancrure 33 de forme quelconque mais de taille adaptée pour laisser passer l'extrémité d'un outil de préhension, ou un doigt d'un manipulateur comme on le voit sur la figure 2, pour saisir le berceau 2 sous-jacent.

Chaque moyen de fermeture 4, 5 est connecté à l'un au moins des volets du berceau 2 de réception (de préférence le volet d'embase 26), et/ou à l'élément de couverture 3.

En particulier, on nomme moyen de fermeture « moteur » un moyen de fermeture qui est connecté au moins au volet de couverture 30 de l'élément de couverture 3. On verra dans la suite qu'un tel moyen de fermeture assure une fonction de transmission d'effort mécanique pendant l'ouverture de l'emballage.

De manière préférée, l'emballage comporte au moins un moyen de fermeture moteur ; le mode de réalisation illustré sur les figures 2 à 9c en comporte deux.

Plus précisément, les moyens de fermeture 4, 5 sont de préférence formés chacun de languettes articulées sur le volet d'embase 26 et le volet de couverture 30, autour de lignes de pliage respectives 42, 52, avantageusement parallèles entre elles et orthogonales aux lignes de pliages 24 et 28 précitées.

Des prolongements 44, 54 de la ligne de pliage longitudinale 24 traversent les moyens de fermeture moteurs 4, 5 divisant ainsi chacun d'entre eux en deux portions, l'une connectée au volet de couverture 30 et l'autre au volet d'embase 26. De manière préférée mais non limitative, chaque moyen de fermeture moteur 4, 5 présente une symétrie par rapport au prolongement 44, 54 de la ligne de pliage longitudinale 24 qui le traverse. Par ailleurs, ces moyens de fermeture 4, 5 peuvent avoir des formes identiques ou distinctes.

Le moyen de fermeture moteur 5 représenté à la droite de la figure 3 s'étend sur un bord transversal complet du volet de couverture 30 et un bord transversal complet du volet d'embase 26 et s'étrécit progressivement en une forme effilée en éloignement de la ligne de pliage 52.

Le moyen de fermeture moteur 4 représenté à gauche de la figure 3 est en forme de languette rectangulaire. Il ne couvre, contrairement au moyen de fermeture 5, qu'une partie d'un bord transversal du volet de couverture 30 et qu'une partie d'un bord transversal du volet d'embase 26, laissant ainsi une portion de bord transversal libre sur le volet d'embase 26 et une portion de bord transversal libre de même longueur sur le volet de couverture 30, les deux portions de bord transversal libres étant opposées par rapport au prolongement 44 de la ligne de pliage longitudinale 24.

En synthèse, l'ébauche représentée sur la figure 3 comprend un volet d'embase 26 de part et d'autre duquel sont articulés respectivement autour des lignes de pliage 28 et 24 le volet de protection 27 pour former le berceau de réception 2 et le volet de couverture 30, ainsi que deux languettes 4, 5 articulées sur des bords transversaux des volets d'embase 26 et de couverture 30 autour de lignes de pliage respectives 42, 52 orthogonales à la ligne de pliage 24.

Vont maintenant être décrites les étapes de pliage de l'emballage 1 selon l'invention, une fois l'ébauche plane représentée sur la figure 3 découpée selon le contour précédemment décrit.

Le volet de protection 27 est d'abord replié selon la ligne de pliage de protection 28 comme illustré en figure 4. L'espace entre le volet d'embase 26 et le volet de protection 27 forme alors un logement 20 conçu pour recevoir le contenu M et l'isoler de l'extérieur.

Le volet de couverture 30 est ensuite replié par rapport à la ligne de pliage longitudinale 24 sur le volet de protection 27. Dans le même mouvement, les moyens de fermeture moteurs sont repliés sur eux-mêmes autour des prolongements 44, 54 de la ligne de pliage longitudinale 24, ce qui leur permet de prendre la forme d'un dièdre 40, 50.

Une fois que le volet de couverture 30 repose contre le volet de protection 27, l'élément de couverture 3 est dans une position repliée dans laquelle les dièdres 40, 50 sont sensiblement plats et l'échancrure 33 sur le volet de couverture 30 laisse découverte une première portion de surface 22 du volet de protection 27, comme illustré sur la figure 6. On notera que le volet de protection 27 est adapté pour couvrir une surface comprenant au moins cette première portion de surface 22 laissée découverte, de manière à prévenir un accès direct au logement 20 depuis l'échancrure 33.

En référence à la figure 7a, les moyens de fermeture 4, 5 sont ensuite repliés selon les lignes de pliages transversales respectives 42, 52 sécantes à la ligne de pliage longitudinale 24 ou même perpendiculaires à celle-ci dans le mode de réalisation illustré comme indiqué précédemment.

On nommera dans la suite ligne de pliage transversale motrice une ligne de pliage transversale liée à un moyen de fermeture moteur. Par exemple, le mode de réalisation illustré comporte deux lignes de pliage transversales 42, 52 toutes deux motrices.

Les moyens de fermeture 4, 5 sont repliés de préférence vers la surface extérieure du volet d'embase 26, soit à l'opposé du volet de couverture 30, selon les lignes de pliage transversales 42, 52 comme illustré en figure 7a, de manière à laisser totalement libre le volet de couverture 30 et par exemple autoriser une grande surface de marquage sur le volet de couverture 30.

La figure 7b représente l'emballage vu de dos alors que les moyens de fermeture 4, 5 sont en train d'être repliés l'un vers l'autre selon leurs lignes de pliage transversales respectives 42, 52. On vient alors engager une portion d'extrémité 46, 56 de l'un des moyens de fermeture dans le dièdre 40, 50 formé par l'autre moyen de fermeture.

Dans le cas où les deux moyens de fermetures 4, 5 forment un dièdre, chaque dièdre peut venir loger au choix dans l'autre dièdre. Sur la figure 7b, c'est le dièdre de droite 44 qui est logé dans le dièdre de gauche 54. L'inverse pourrait être opéré en alternative.

En référence à la figure 8a, les moyens de fermeture 4, 5 reposent contre la surface extérieure du volet d'embase 26, et sont maintenus ensemble uniquement grâce à cet engagement mécanique qui maintient l'emballage 1 clos.

On notera que la forme des moyens de fermeture 4, 5 est adaptée pour satisfaire deux exigences.

Premièrement, leurs longueurs respectives dans la direction de la ligne de pliage longitudinale 24 doivent être suffisantes pour réaliser l'engagement de l'un dans l'autre.

Par exemple, dans le cas où les lignes de pliage transversales 42, 52 sont perpendiculaires à la ligne de pliage longitudinale 24, la somme des longueurs des moyens de fermeture 4, 5 doit être plus grande que la longueur de la ligne de pliage longitudinale 24.

Deuxièmement, les moyens de fermetures 4, 5 maintenus ensemble doivent laisser découverte au moins une deuxième portion de surface 22' du berceau 2 de réception, localisée sur le volet d'embase 26, de sorte que la préhension du berceau de réception 2 laisse libre les moyens de fermeture 4, 5. Cette deuxième portion découverte 22' contient de préférence une portion symétrique par rapport à la ligne de pliage de protection 28 à la portion 22 du volet de protection 27 laissée découverte grâce à l'échancrure 33.

De manière préférée, lorsque les deux moyens de fermeture 4, 5 sont moteurs, les lignes de pliage transversales 42, 52 sont choisies perpendiculaires à la ligne de pliage longitudinale 24. Ainsi, lorsque les dièdres 40, 50 sont engagés l'un dans l'autre, leurs arêtes respectives sont en contact sur au moins un segment qui améliore la rigidité de fixation des moyens de fermeture 4, 5.

L'on peut prévoir de placer le contenu M dans l'emballage au cours du processus de pliage précité, typiquement avant l'étape de pliage du volet de couverture 30 illustré en figure 6, ou de procéder à une phase de mise en forme complète de l'emballage par pliage comme illustré sur les figures 2 à 8 puis de rouvrir l'emballer afin d'y introduire le contenu M avant de refermer l'emballage.

L'ouverture de l'emballage ainsi préparé va maintenant être détaillée au regard des figures 2 et 9a à 9c.

En référence à la figure 2, un manipulateur saisit l'emballage manuellement.

Une première main pince le berceau 2 de réception sur les portions laissées découvertes, par exemple son index sur la portion découverte 22' du volet d'embase 26 laissée par les dièdres 40, 50 et son pouce sur la portion découverte 22 du volet de protection 27 laissée par l'échancrure 33.

L'autre main peut ensuite saisir le bord libre 35 du volet de couverture 30 et le solliciter en éloignement du berceau de réception 2 pour soulever le volet de couverture 30. Pour assurer sa prise, le manipulateur peut glisser son pouce entre le volet de couverture 30 et le volet de protection 27 sous-jacent et ainsi pincer une zone de préhension 32 du volet de couverture 30 adjacente au bord libre 35.

On notera que la portion de bord transversal libre 34, présente sur le volet de couverture 30, autorise un pelage de la zone de préhension 35 sans déclencher immédiatement l'ouverture de l'emballage 1, puisque tant que la sollicitation du volet de couverture 30 est limitée à cette portion libre 34 du bord transversal, aucune sollicitation n'est transmise aux moyens de fermeture 4, 5.

Sur la figure 9a, les doigts du manipulateur ne sont pas représentés. Le bord libre 35 du volet de couverture 30 est pelé à gauche de l'échancrure 33, la portion de bord transversal libre 34 étant située sur le bord gauche du volet de couverture 30.

Lorsque le manipulateur soulève le volet de couverture 30, la zone de préhension 32 se tend et applique une contrainte de flexion sur chaque lignes de pliage transversale motrice 42, 52. Cette contrainte de flexion fait subir à chaque moyen de fermeture moteur 4, 5 une déformation de son dièdre 40, 50 à la fois dans le sens d'une ouverture autour des lignes de pliage 44, 54 et de pivotement autour des lignes de pliage 42, 52, qui déclenche son dépliage automatique selon la ligne de pliage transversale motrice 42, 52 qui lui est associée. Au-delà d'un angle limite de rotation d'un moyen de fermeture moteur autour de sa ligne de pliage transversale motrice 42, 52, l'autre moyen de fermeture ne loge plus dans son dièdre; l'engagement mécanique des moyens de fermetures 4, 5 entre eux est alors rompu.

Le dépliage de chaque moyen de fermeture moteur 4, 5 selon sa ligne de pliage transversale motrice 42, 52 se poursuit jusqu'à ce que le prolongement 44, 54 qui le traverse soit au moins sensiblement aligné avec la ligne de pliage longitudinale 24, comme illustré sur la figure 9c. Comme indiqué précédemment, le soulèvement du volet de couverture 30 amorce le dépliage de l'élément de couverture 3 selon la ligne de pliage longitudinale 24, qui entraîne le dépliage de chaque dièdre 40, 50 selon sa ligne de pliage 44, 54.

Une fois le dépliage de l'élément de couverture 3 terminé, le manipulateur a toujours une main qui pince le berceau 2 de protection maintenant découvert ; il peut présenter l'emballage 1 ouvert à un tiers pour que celui-ci puisse saisir le contenu M logé dans le logement 20 à l'aide de moyens appropriés, tels qu'une pince stérile. Alternativement, le manipulateur peut lui-même procéder à l'extraction du contenu M avec sa main libre.

Pour faciliter l'extraction du contenu M par un tiers, le manipulateur peut éventuellement appliquer avec sa main libre une légère flexion au volet de couverture 30, à l'un des moyens de fermeture 4, 5 ou au volet de protection 27, de manière à libérer un accès plus large au logement 20.

### VARIANTES

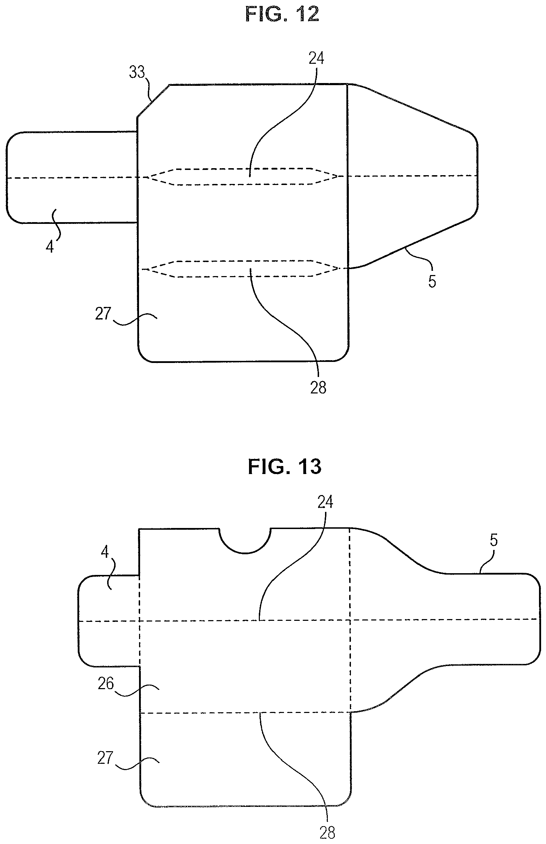

Des variantes de réalisation portant sur des caractéristiques spécifiques de l'emballage vont maintenant être décrites en regard des figures 10 à 17. Ces variantes peuvent toute être combinées entre elles.
- Dans le mode de réalisation illustré sur les figures 3 à 8, les deux moyens de fermeture 4, 5 sont moteurs. En variante illustré sur la figure 10, l'emballage 1 comporte un moyen de fermeture moteur 5 et un autre moyen de fermeture 4 connecté uniquement au volet d'embase 26. A la fermeture de l'élément de couverture 3, seul le moyen de fermeture moteur 5 forme un dièdre, tandis que le moyen de fermeture 4 non moteur conserve une forme plane engagée par la suite à l'intérieur du dièdre du moyen de fermeture moteur 5. Le moyen de fermeture 4 non moteur n'assure pas de fonction de transmission d'effort mécanique lors de l'ouverture de l'emballage : son dépliage suivant sa ligne de pliage transversale 42 est mécaniquement entraîné par le dépliage du moyen de fermeture moteur 5 dans lequel il loge.
- Comme illustré figure 11, l'emballage conforme à la présente invention n'est pas limité à un contour rectangulaire. Le volet d'embase 26 peut par exemple être globalement en forme de parallélogramme, les deux volets de protection 27 et de couverture 30 étant articulés sur le volet d'embase 26 autour des lignes de pliage 24, 28 non parallèles mais restant globalement symétriques du volet d'embase 26 par rapport à ces lignes de pliages 24, 28.
- Comme illustré figure 12, l'une au moins des lignes de pliage 24, 28 peut être double sur au moins une partie de sa longueur pour permettre de donner de l'épaisseur à l'emballage, notamment au berceau 2 une fois l'emballage mis en forme.
- Par ailleurs les moyens mécaniques de maintien sans collage, formés d'au moins un dièdre selon le mode de réalisation précédemment décrit, peuvent être remplacés par tout moyen mécanique équivalent, par exemple la réalisation d'au moins une incision longitudinale 55 débouchant sur le bord libre d'un moyen de fermeture 5 opposé à la ligne d'articulation 52 afin de recevoir l'extrémité du moyen de fermeture complémentaire 4 comme illustré figures 14a et 14b.
- Les moyens de fermeture 4, 5 peuvent être repliés vers l'arrière contre le volet d'embase 26, comme illustré en figure 7a, ou en variante, vers l'avant contre le volet de couverture 30 de l'élément de couverture 3, comme illustré sur la figure 15. La surface de marquage sur le volet de couverture 30 est alors diminuée, mais le manipulateur peut surveiller directement la séparation des moyens de fermetures 4, 5 et donc avoir un meilleur contrôle visuel de l'ouverture de l'emballage 1. Par ailleurs dans ce cas un marquage peut être opéré sur le volet d'embase 26.
- L'échancrure 33 peut être placée sensiblement au milieu du bord libre 35, comme illustré sur les figures 2 à 9. En variante, l'échancrure 33 est placée à l'angle d'un bord transversal et du bord libre 35 de l'élément de couverture 3, comme illustré sur les figures 12 et 16. Ainsi, les portions de surface 22, 22' laissées découvertes sur le berceau de réception 2 sont situées dans un coin du volet de protection 27 et du volet d'embase 26. Selon une autre variante illustrée figure 17 le bord libre 35 du volet de couverture 30 peut posséder une concavité 33 qui s'étend continûment entre ses deux extrémités.
- Le trou laissé par l'échancrure 33 illustré sur les figures 2 à 9 est semi-circulaire. En variante, ce trou peut être de forme polygonale (un trapèze sur les figures 10 et 11, un triangle sur la figure 12), ovoïde, ou de toute autre forme appropriée.
- Les moyens de fermetures 4, 5 peuvent présenter des longueurs selon la direction de leurs lignes de pliage 44, 54 sensiblement identiques, comme illustré sur la figure 3, ou bien différentes, comme on peut le voir sur la variante de réalisation illustrée en figure 13.
- le volet de protection 27 peut posséder une largeur, considérée perpendiculairement à la ligne de pliage de protection 28, sensiblement égale à celle du volet d'embase 26 comme illustré sur la figure 3, de manière à venir le recouvrir totalement par pliage selon la ligne 28. En variante illustré en figure 12, cette largeur du volet de protection 27 peut être inférieure à celle du volet d'embase 26, tout en étant supérieure à la profondeur de l'échancrure 33 du volet de couverture 30 de manière à prévenir tout accès au logement 20 du berceau 2 lorsque l'emballage est en position repliée.
- Les moyens de fermetures étrécis progressivement en une forme effilée en éloignement de la ligne de pliage 52 peuvent présenter un étrécissement curviligne, comme illustré en figure 3, ou droit, comme illustré en figure 12.

### MATERIAUX UTILISES

La feuille de l'emballage est réalisée dans un matériau adapté pour héberger le contenu M, par exemple un contenu de type mèche de gaze ou tricot. On peut donc utiliser un matériau de grade médical, compatible avec différents procédés de stérilisation. Ce matériau doit protéger le contenu M mécaniquement (manipulations, humidité), assurer une barrière microbiologique afin de maintenir la stérilité du contenu M et générer le moins possible de particules.

Pour améliorer la résistance des portions de l'emballage mises sous contrainte lors de son ouverture, le matériau est de préférence indéchirable, et plus souple que du papier. En outre, ce matériau est préférablement recyclable. Très préférentiellement, ce matériau est un matériau textile non-tissé de fibres, par exemple en polyéthylène haute densité. Un tel matériau est connu sous le nom « Tyvek » (marque déposée). Un avantage procuré par un matériau textile est qu'il peut présenter une rigidité suffisante pour assurer le maintien des moyens de fermetures 4, 5 engagés l'un dans l'autre et présenter une glisse qui facilite l'ouverture.

## Revendications

1. Emballage (1) pour matériel médical réalisé par pliage d'une feuille de sorte à former un berceau (2) de réception du contenu (M), formé de deux volets (26, 27) articulés entre eux en forme de pince, un élément de couverture (3) articulé sur l'un des volets du berceau (2) et des moyens de fermeture (4, 5) eux-mêmes articulés transversalement sur l'un au moins des volets (26, 27, 30) du berceau (2) et/ou de l'élément de couverture (3), **caractérisé par le fait que** les moyens de fermeture (4, 5) sont adaptés pour être fixés simplement par engagement mécanique entre eux et que l'élément de couverture (3) exerce, lors de sa sollicitation à l'ouverture, un effort sur lesdits moyens de fermeture (4, 5) engagés mécaniquement entre eux, assurant leur séparation automatique et de là l'ouverture de l'emballage (1).

2. Emballage (1) selon la revendication 1, **caractérisé en ce qu'**au moins un des moyens de fermeture (4, 5) est articulé transversalement sur l'élément de couverture (3) par une ligne de pliage transversale (42, 52).

3. Emballage (1) selon l'une des revendications 1 ou 2, **caractérisé en ce qu'**au moins un des moyens de fermeture (4, 5) est un moyen de fermeture moteur articulé transversalement sur un des volets du berceau (2) et sur l'élément de couverture (3) par une ligne de pliage transversale motrice (42, 52).

4. Emballage (1) selon la revendication 3, **caractérisé en ce que** l'élément de couverture (3) est articulé à un volet d'embase (26) du berceau par une ligne de pliage longitudinale (24) sécante avec chaque ligne de pliage transversale (42, 52).

5. Emballage (1) selon la revendication 4, **caractérisé en ce que** chaque moyen de fermeture moteur (4, 5) est traversé par un prolongement (44, 54) de la ligne de pliage longitudinale (24) de l'élément de couverture (3) sur le volet d'embase (26), lui permettant de former un dièdre (40, 50) lorsque l'élément de couverture (3) est en position repliée.

6. Emballage (1) selon l'une des revendications 1 à 5, **caractérisé en ce qu'**il comporte deux moyens de fermeture (4, 5) dont le premier au moins est un moyen de fermeture moteur en forme de dièdre (40, 50), l'engagement mécanique entre eux dans leur position repliée étant réalisé par logement d'une portion (46, 56) du second moyen de fermeture à l'intérieur du dièdre (40, 50) formé par le premier moyen de fermeture moteur.

7. Emballage (1) selon l'une des revendication 4 à 5, caractérisé en ce les deux moyens de fermetures (4, 5) sont moteurs et en forme de dièdre (40, 50), que les deux lignes de pliage transversales motrices (42, 52) sont perpendiculaires à la ligne de pliage longitudinale (24) de l'élément de couverture (3) sur le volet d'embase (26), et que les arêtes des dièdres (40, 50) se touchent en au moins un segment lorsque les moyens de fermeture (4, 5) sont engagés l'un dans l'autre dans leur position repliée.

8. Emballage (1) selon l'une des revendications 1 à 7, **caractérisés en ce que** l'élément de couverture (3) comporte un volet de couverture (30) comprenant un bord libre (35) opposé à une ligne de pliage longitudinale (24), dans lequel est formée au moins une échancrure (33) laissant découverte une portion de surface (22) du berceau (2) lorsque l'élément de couverture (3) est en position repliée.

9. Emballage (1) selon l'une des revendications 1 à 8, **caractérisé en ce que** l'élément de couverture (3) comporte un volet de couverture (30) comprenant au moins un bord transversal comportant une portion transversale connectée à un moyen de fermeture et une portion transversale libre (34) adjacente à un bord libre (35).

10. Emballage (1) selon l'une des revendications 1 à 9, **caractérisé en ce que** les moyens de fermeture (4, 5) sont repliés contre le berceau (2) et le recouvrent partiellement de sorte à laisser découverte une portion de surface (22') sur le berceau (2).

11. Emballage (1) selon les revendications 8 et 10 prises en combinaison, **caractérisé en ce que** le berceau (2) comprend un volet de protection (27) replié sur un volet d'embase (26) par une ligne de pliage de protection (28), les deux portions de surface (22, 22') laissées découvertes sur le berceau (2) s'étendant de part et d'autre de cette ligne de pliage de protection (28) de sorte à pouvoir être saisies simultanément par pinçage.

12. Emballage (1) selon l'une des revendications 1 à 11, **caractérisé en ce qu'**au moins un moyen de fermeture moteur (4, 5) est symétrique par rapport au prolongement (44, 54) d'une ligne de pliage longitudinale (24) qui le traverse, de manière à faire coïncider les deux faces du dièdre (40, 50) qu'il forme lorsque l'élément de couverture (3) est en position repliée.

13. Emballage (1) selon l'une des revendications 1 à 12, réalisé en un matériau textile non-tissé, de préférence en fibres de polyéthylène.

## Patentansprüche

1. Verpackung (1) für medizinisches Gerät, hergestellt durch Falten eines Blattes, um eine Mulde (2) zur Aufnahme des Inhalts (M) zu formen, aus zwei Teilen (26, 27) geformt, die gegenseitig zangenförmig angelenkt sind, einem Abdeckelement (3), das an einem der Teile der Mulde (2) angelenkt ist, und Schließmitteln (4, 5), die selbst querlaufend auf zumindest einem der Teile (26, 27, 30) der Mulde (2), und/ oder des Abdeckelements (3) angelenkt sind, **dadurch gekennzeichnet, dass** die Schließmittel (4, 5) ausgeführt sind, um einfach durch mechanisches Eingreifen ineinander befestigt zu werden, und das Abdeckelement (3) bei seiner Beaufschlagung zum Öffnen eine Kraft auf die besagten Schließmittel (4, 5) ausübt, die mechanisch ineinander eingreifen, wodurch für deren automatische Trennung, und dadurch für die Öffnung der Verpackung (1) gesorgt wird.

2. Verpackung (1) nach Anspruch 1, **dadurch gekennzeichnet, dass** zumindest eines der Schließmittel (4, 5) querlaufend durch eine querlaufende Faltungslinie (42, 52) auf dem Abdeckelement (3) angelenkt ist.

3. Verpackung (1) nach einem der Ansprüche 1 oder 2, **dadurch gekennzeichnet, dass** zumindest eines der Schließmittel (4, 5) ein treibendes Schließmittel ist, das querlaufend auf den Teilen der Mulde (2) und auf dem Abdeckelement (3) durch eine treibende querlaufende Faltungslinie (42, 52) angelenkt ist.

4. Verpackung (1) nach Anspruch 3, **dadurch gekennzeichnet, dass** das Abdeckelement (3) durch eine längslaufende Faltungslinie (24) an einem Fußflächenteil (26) der Mulde angelenkt ist, die jede querlaufende Faltungslinie (42, 52) schneidet.

5. Verpackung (1) nach Anspruch 4, **dadurch gekennzeichnet, dass** jedes treibende Schließmittel (4, 5) durch eine Verlängerung (44, 54) der längslaufenden Faltungslinie (24) des Abdeckelements (3) auf dem Fußflächenteil (26) gequert wird, wodurch es ihm ermöglicht wird, ein Dieder (40, 50) zu formen, wenn das Abdeckelement (3) in der gefalteten Position ist.

6. Verpackung (1) nach einem der Ansprüche 1 bis 5, **dadurch gekennzeichnet, dass** sie zwei Schließmittel (4, 5) umfasst, von denen zumindest das erste ein treibendes Schließmittel in Form eines Dieders (40, 50) ist, wobei das mechanische Eingreifen ineinander in ihrer gefalteten Position durch die Aufnahme eines Abschnitts (46, 56) des zweiten Schließmittels im Inneren des Dieders (40, 50) erfolgt, das durch das erste treibende Schließmittel geformt wird.

7. Verpackung (1) nach einem der Ansprüche 4 bis 5, **dadurch gekennzeichnet, dass** die beiden Schließmittel (4, 5) treibend, und in Form eines Dieders (40, 50) sind, die beiden treibenden querlaufenden Faltungslinien (42, 52) senkrecht zu der längslaufenden Faltungslinie (24) des Abdeckelements (3) auf dem Fußflächenteil (26) sind, und sich die Kanten der Dieder (40, 50) in zumindest einem Segment berühren, wenn die Schließmittel (4, 5) in ihrer gefalteten Position ineinander eingreifen.

8. Verpackung (1) nach einem der Ansprüche 1 bis 7, **dadurch gekennzeichnet, dass** das Abdeckelement (3) ein Abdeckteil (30) umfasst, das einen freien Rand (35) gegenüber einer längslaufenden Faltungslinie (24) enthält, in dem zumindest eine Aussparung (33) geformt ist, die einen Oberflächenabschnitt (22) der Mulde (2) abgedeckt lässt, wenn das Abdeckelement (3) in der gefalteten Position ist.

9. Verpackung (1) nach einem der Ansprüche 1 bis 8, **dadurch gekennzeichnet, dass** das Abdeckelement (3) ein Abdeckteil (30) umfasst, das zumindest einen querlaufenden Rand umfasst, der einen querlaufenden Abschnitt umfasst, der mit einem Schließmittel verbunden ist, und einen freien querlaufenden Abschnitt (34) angrenzend an einen freien Rand (35).

10. Verpackung (1) nach einem der Ansprüche 1 bis 9, **dadurch gekennzeichnet, dass** die Schließmittel (4, 5) gegen die Mulde (2) gefaltet sind, und sie teilweise verdecken um einen Oberflächenabschnitt (22') auf der Mulde (2) offengelegt zu lassen.

11. Verpackung (1) nach den Ansprüchen 8 und 10, in Kombination miteinander, **dadurch gekennzeichnet, dass** die Mulde (2) ein Schutzteil (27) umfasst, das durch eine Schutzfaltungslinie (28) auf ein Fußflächenteil (26) gefaltet ist, wobei sich die beiden auf der Mulde (2) offengelegt gelassenen Oberflächenabschnitte (22, 22') beiderseits dieser Schutzfaltungslinie (28) erstrecken, um gleichzeitig durch Kneifen ergriffen werden zu können.

12. Verpackung (1) nach einem der Ansprüche 1 bis 11, **dadurch gekennzeichnet, dass** zumindest ein treibendes Schließmittel (4, 5) im Verhältnis zur Verlängerung (44, 54) einer längslaufenden Faltungslinie (24) symmetrisch ist, die sie quert, um die beiden Flächen des Dieders (40, 50), das sie formt zusammenfallen zu lassen, wenn das Abdeckelement (3) in der gefalteten Position ist.

13. Verpackung (1) nach einem der Ansprüche 1 bis 12, die aus einem Vliesstoff, vorzugsweise aus Polyethylenfasern hergestellt ist.

## Claims

1. A packaging (1) for an item of medical equipment produced by folding a sheet so as to form a cradle (2) for receiving the content (M), made of two flaps (26, 27) hinged together in the shape of a clamp, a cover element (3) hinged on one of the flaps of the cradle (2) and closing means (4, 5) themselves hinged transversely on at least one of the flaps (26, 27, 30) of the cradle (2) and/or the cover element (3), **characterized by** the fact that the closing means (4, 5) are designed to be fixed together by simple mechanical engagement between them and that, when urged to open, the cover element (3) exerts a force on said closing means (4, 5) mechanically engaged with each other, making them automatically separate and thus opening the packaging (1).

2. The packaging (1) according to claim 1, **characterized in that** at least one of the closing means (4, 5) is hinged transversely on the cover element (3) by a transverse fold line (42, 52).

3. The packaging (1) according to one of claims 1 or 2, **characterized in that** at least one of the closing means (4, 5) is a driving closing means hinged transversely on one of the flaps of the cradle (2) and on the cover element (3) by a driving transverse fold line (42, 52).

4. The packaging (1) according to claim 3, **characterized in that** the cover element (3) is hinged to a base flap (26) of the cradle by a longitudinal fold line (24) secant with each transverse fold line (42, 52).

5. The packaging (1) according to claim 4, **characterized in that** each driving closing means (4, 5) is traversed by an extension (44, 54) of the longitudinal fold line (24) of the closing element (3) on the base flap (26), allowing it to form a dihedron (40, 50) when the cover element (3) is in the folded position.

6. The packaging (1) according to one of claims 1 to 5, **characterized in that** it includes two closing means (4, 5) at least the first of which is a driving closing means in the shape of a dihedron (40, 50), the mechanical engagement between them in their folded position being effected by encasing a section (46, 56) of the second closing means within the dihedron (40, 50) formed by the first driving closing means.

7. The packaging (1) according to one of claims 4 to 5, **characterized in that** both closing means (4, 5) are driving and in the shape of a dihedron (40, 50), that both driving transverse fold lines (42, 52) are perpendicular to the longitudinal fold line (24) of the cover element (3) on the base flap (26), and that the edges of the dihedrons (40, 50) touch each other in at least one segment when the closing means (4, 5) are engaged inside each other in their folded position.

8. The packaging (1) according to one of claims 1 to 7, **characterized in that** the cover element (3) includes a covering flap (30) comprising a free edge (35) opposite a longitudinal fold line (24), wherein is formed at least one notch (33) leaving a section of the surface (22) of the cradle (2) uncovered, when the cover element (3) is in the folded position.

9. The packaging (1) according to one of claims 1 to 8, **characterized in that** the cover element (3) includes a covering flap (30) comprising at least one transverse edge including a transverse section connected to a closing means and a free transverse section (34) adjacent to a free edge (35).

10. The packaging (1) according to one of claims 1 to 9, **characterized in that** the closing means (4, 5) are folded down against the cradle (2) and partly cover it in such a way as to leave a section of the surface (22') on the cradle (2) uncovered.

11. The packaging (1) according to claims 8 and 10 taken together, **characterized in that** the cradle (2) comprises a protective flap (27) folded on a base flap (26) by a protective fold line (28), the two surface sections (22, 22') left uncovered on the cradle (2) extending on either side of this protective fold line (28) so as to be simultaneously taken hold of in a clamping movement.

12. The packaging (1) according to one of claims 1 to 11, **characterized in that** at least one driving closing means (4, 5) is symmetrical with respect to the extension (44, 54) of a longitudinal fold line (24) that traverses it, in such a way that the two faces of the dihedron (40, 50) that it forms when the cover element (3) is in the folded position coincide.

13. The packaging (1) according to one of claims 1 to 12, made of a non-woven textile material, preferably polyethylene fibers.
